# EUROPEAN PATENT SPECIFICATION

(11) **EP 0 305 097 B1**
(45) Date of publication and mention of the grant of the patent: **27.10.1993**
(21) Application number: 88307554.1
(22) Date of filing: 15.08.1988
(51) Int. Cl.: A61K 33/00, A61K 31/19

(54) **Nutritional supplement**
Nahrungsmittelzusatz
Supplément nutritif

(30) Priority: 25.08.1987 GB 8719988
(43) Date of publication of application: 01.03.1989
(60) Divisional application: 90123713.1
(73) Proprietor: SCOTIA HOLDINGS PLC, Guildford Surrey GU1 1BA (GB)
(72) Inventor: Horrobin, David Frederick, Halesmere Surrey (GB)
(74) Representative: Cockbain, Julian, Dr.

(56) References cited:
- EP-A- 0 192 367
- WO-A-86/00810
- BE-A- 886 050
- Pharmacological Basis of Therapeutics, Ed. 7, A. Goodman-Gilman et al., MacMillan, 1985, New York, US
- Litium in Biology and Medicine, VCH Verlag, Weinheim, New York, Edited by G.N. Schrauzer and K.-F. Klippel (1991), pp. 149-166

## Description

The present invention relates to the use of physiologically tolerable lithium compounds and in particular to the use of such compounds for the manufacture of nutritional supplements for use in combatting conditions associated with essential chemical deficiency.

Lithium compounds have been widely used in the treatment of manic depressive psychosis but have also been reported as being suitable for the treatment of other ailments. Thus Sherwin in US- A-3639625 suggests the use of topical lithium succinate compositions for the treatment of various skin diseases, Horrobin and Lieb in US-A-4386072 suggest the use of lithium compounds for the treatment of certain disorders of inflammation and immunity, EP-B-85579 (Efamol Ltd.) suggests the use of lithium compounds for the treatment of skin lesions, and EP-A-132126 (Efamol Ltd.) suggests the use of topical lithium-containing compositions in the treatment of seborrheic dermatitis, a fungal disease arising from excessive multiplication of a fungus which is normally present on the skin surface at tolerable levels.

BE-A-886050 describes orally administrable lithium containing solutions which function to improve blood circulation, especially to the brain. The lithium is described however as being present only to aid the distribution of trace elements present in the solution and not as having a physiological function itself.

Lithium treatment however has often been accompanied by a number of side-effects which are generally related to dosage levels and to the degree of accumulation. Particular side-effects which have occurred, especially in the treatment of manic depressive illness, include transient nausea, fine tremor, fatigue, muscular weakness, polydipsia and polyuria. Thus it is stated in Martindale The Extra Pharmacopoeia 28th Edition (1982) 1538 that the margin between the therapeutic and the toxic concentration of lithium is narrow, and that therefore it should be given under close medical supervision.

We have now surprisingly found that significantly depressed lithium plasma levels, relative to those of healthy subjects, are associated with a number of ailments and diseases, in other words that lithium appears to be an essential trace element.

Thus in healthy subjects lithium plasma levels may generally be in excess of 0.04 mM/l, whereas in subjects suffering from atopic eczema or seborrheic dermatitis mean plasma levels of below 0.025 mM/l are found. Alcoholics, patients with psoriasis, candidiasis, pityriasis and other fungal skin infections and sufferers from combination skin similarly exhibit depressed lithium plasma levels. (Combination skin is a troublesome and unsightly complaint manifested by excessive greasiness in certain skin areas, such as the forehead and the nose, and excessive dryness in other skin areas, such as the sides of the face).

We now propose therefore that conditions associated with essential chemical deficiency due to low lithium plasma levels may be countered or prevented from arising by the administration of a nutritional supplement containing a physiologically tolerable lithium compound.

Viewed from one aspect, the present invention thus provides the use of a physiologically tolerable lithium compound for the manufacture of a nutritional supplement for use in combatting conditions associated with lithium deficiency in humans, said nutritional supplement being in the form of a complete foodstuff or a multi-vitamin/multi-mineral preparation.

The nutritional supplement, which conveniently may be sterile, may be in a form adapted for enteral, parenteral or topical administration, but most preferably will be in a form adapted for oral ingestion or parenteral, e.g. iv, injection or infusion. Liquid preparations made with sterile or deionized water are particularly preferred. However, in another preferred embodiment the nutritional supplement may take the form of dietary supplement, such as a foodstuff. The nutritional supplement may alternatively be in a conventional pharmaceutical dosage form adapted for administration to the gastrointestinal tract. In this regard, forms such as tablets, coated tablets, capsules, powders, drops, suspensions, solutions, syrups and suppositories deserve particular mention. Nevertheless as lithium nutritional supplementation may be achieved by parenteral administration, for example by injection, the nutritional supplement may be in the form of a composition adapted for one of these administration modes.

Where the nutritional supplement is prepared in a conventional pharmaceutical dosage form it may of course also contain conventional pharmaceutical carriers or excipients.

For parenteral administration by injection or infusion the nutritional supplements of the invention are preferably formulated as sterile solutions, emulsions or suspensions, e.g. in water for injections or in solutions in a lipid or lipid solvent, e.g. the alcohol analogues of the polyunsaturated fatty acids discussed below.

For oral administration, the nutritional supplement may conveniently take the form of a foodstuff, for example a food or drink mix, into which a lithium compound is incorporated. The lithium containing nutritional supplement of the invention may particularly suitably be in the form of a so-called "complete" foodstuff, analogous to those which are prepared to serve as the major or sole source of nutrition for example for people wishing to loose weight, for post-operative patients, for elderly patients, for convalescents, or for individuals with specific dietary needs (e.g. patients with diabetes, coeliac disease or cystic fibrosis). For most people however a lithium supplemented foodstuff will preferably be of a type that is ingested daily in similar quantities, and for this reason the supplement will particularly conveniently comprise the lithium compound and a cereal or legume foodstuffs base. In an especially preferred embodiment, the nutritional supplement may be in the form of a breakfast cereal. In a preferred alternative, however, the nutritional supplement may take the form of a lithium-containing multi-vitamin/multi-mineral preparation, for example in the form of tablets, capsules, or drops. In this regard, compositions are especially preferred which contain the lithium compound together with sources of one, some or all of the vitamins and the other essential elements, for example selected from vitamins A, B₁, B₂, B₃, B₆, B₁₂, C, D and E and calcium, copper, zinc, manganese and iron. In another embodiment, the lithium compound may be incorporated into an enteral or parenteral alimentation solution. The nutritional supplement of the invention may however contain the lithium compound as the sole active ingredient.

The lithium compound used according to the invention may be any physiologically tolerable lithium compound that permits lithium uptake by the body. In general therefore, the preferred compounds will be lithium salts and in particular those salts which are at least sparingly water-soluble. Particularly suitable lithium compounds thus include lithium carbonate, lithium citrate, lithium chloride, lithium succinate, lithium sulphate, lithium salicylate, lithium acetylsalicylate, lithium orotate and the lithium salts of polyunsaturated fatty acids, especially the C₁₈₋₂₂ fatty acids and more especially the essential fatty acids. Thus it is especially preferred that the nutritional supplement contain at least one lithium salt of an essential fatty acid in the n-6 series (e.g. 18:2 n-6 (linoleic acid), 18:3 n-6 (gammalinolenic acid), 20:3 n-6 (dihomogammalinolenic acid), 20:4 n-6 (arachidonic acid), 22:4 n-6 (adrenic acid) and 22:5 n-6) or in the n-3 series (e.g. 18:3 n-3 (alpha linolenic acid), 18:4 n-3, 20:4 n-3, 20:5 n-3 (eicosapentaenoic acid), 22:5 n-3 and 22:6 n-3 (docosahexaenoic acid)).

Most especially preferably the nutritional supplement will contain at least one essential fatty acid of the n-3 series, or a salt, most preferably the lithium salt thereof, and at least one essential fatty acid of the n-6 series, or a salt, again most preferably the lithium salt, thereof.

The essential fatty acids are essential nutrients which must be provided in the diet because they cannot be manufactured by the body. The main dietary essential fatty acids are linoleic and alphalinolenic acids but to be fully utilised the essential fatty acids must be metabolised along the n-3 or n-6 pathways, of which alphalinolenic and linoleic acids are the respective starting points.

In patients who are receiving no food or only insufficient food by the normal oral route the essential fatty acids must be provided by enteral or parenteral feeding.

On enteral adminstration, however, the fatty materials may not be fully absorbed by patients having fat malabsorbtion. Such malabsorbtion is particularly likely to occur with patients with defective pancreatic or hepatic functions, with certain intestinal diseases or with cystic fibrosis. Essential fatty acids thus may be administered intravenously as parenteral nutrition supplements but at present this is generally done by administration of liquid emulsions, usually containing linoleic acid with or without alphalinolenic acid. Such liquid emulsions are not readily compatible with conventional aqueous fluids for parenteral nutrition, administration of liquid emulsions is not clear of side effects and there is particular concern regarding possible effects on the lungs, especially of infants.

We have found that the lithium salts of the essential fatty acids surprisingly are crystalline solids at ambient temperature which are highly soluble in water and alcohol. These properties facilitate preparation of compositions containing lithium or the essential fatty acids and thus the salts can be used to promote lithium and/or essential fatty acid uptake following administration. Where essential fatty acids are to be incorporated into the nutritional supplements of the invention they may accordingly be particularly conveniently incorporated as the lithium salts. However the essential fatty acids may also conveniently be incorporated in the form of salts with other counterions, especially the sodium and potassium salts which are also water soluble. Particularly conveniently, the nutritional supplement may contain two or more essential fatty acid sodium, lithium or potassium salts.

Where the nutritional supplements are for parenteral administration and contain essential fatty acid salts they are preferably formulated as aqueous solutions, and preferably are stored under an inert atmosphere, e.g. in nitrogen flushed vials, buffered to a pH in the range 8.5 to 9.0, e.g. with a physiologically acceptable acid such as citric acid. Moreover, such solutions preferably contain an antioxidant, e.g. a fatty acid derivative of ascorbic acid such as ascorbyl palmitate, a tocopherol (e.g. gamma tocopherol or a mixed tocopherol) or butylated hydroxy toluene.

The essential fatty acid supply required to prevent essential fatty acid deficiency is probably within the range 0.5-8% of total daily calorie intake, especially 1-3% (or 1-5% during periods of metabolic stress such as rapid growth, pregnancy, lactation or injury). In the case of orally or parenterally administrable nutritional supplements, the nutritional supplements are preferably administered at a daily or one-off dosage of the essential fatty acid or salt thereof of 1 to 100,000 mg, especially 1-50000 mg, preferably 100-10000 mg, conveniently in dosage units of 50, 100, 250, 500 or 1000 mg. For topical administration, concentrations of the essential fatty acids or salts thereof may conveniently be 0.001 to 50%, for example from 0.01 to 30%, preferably 0.1 to 5%, by weight.

Where the nutritional supplement of the invention contains a lithium salt of a polyunsaturated fatty acid and is in a form adapted for oral administration, the lithium salt in the supplement is preferably provided with a gastric juice resistant release delaying coating, e.g. a Eudragit^{R} coating as supplied by Röhm GmbH of Darmstadt.

The lithium content in the nutritional supplement will be selected according to the nature of the supplement and its administration route but in general will be within the range 1 ppb to 30% by weight of lithium, preferably 1 ppm to 20% lithium, especially preferably 0.001 to 10% lithium and particularly preferably up to 1% lithium. Obviously, where the nutritional supplement is in the form of a complete foodstuff, the lithium content will be towards the lower ends of the ranges specified above, for example it may conveniently be in the range 1 ppb to 10 ppm, preferably 10 ppb to 1 ppm. Thus, for example in complete foods which might be administered in a dose of 500g/day, a lithium content of 5mg might be contemplated. More particularly, the daily dosage will generally be such that the adult body receives from 1 µg to 50 mg, preferably 1 to 10 mg, of lithium per day, and it will preferably be such as to maintain the lithium plasma level above 0.04 mM/l.

Preferably the nutritional supplement comprises a sterile foodstuffs base with included therein a physiologically tolerable lithium compound in a concentration such that said supplement contains from 1 ppb to 1% by weight of lithium.

The nutritional supplement may conveniently comprise a sterile composition containing a physiologically tolerable lithium compound together with four or more, preferably eight or more, vitamins or minerals, for example essential vitamins or minerals selected from the group consisting of vitamins A, B₁, B₂, B₃, B₆, B₁₂, C, D and E and physiologically tolerable calcium, copper, zinc, manganese and iron compounds, and optionally together with at least one physiologically acceptable carrier or excipient.

Alternatively, as mentioned above the nutritional supplement may take the form of a sterile parenteral or enteral alimentation solution containing a physiologically tolerable lithium compound.

The nutritional supplement may be used to combat a wide range of conditions associated with essential chemical deficiency, in particular conditions which appear to be associated with immune system malfunction and especially conditions such as atopic eczema, psoriasis, seborrheic dermatitis, candidiasis, pityriasis, skin fungal infections and conditions associated with alcoholism.

The present invention will now be illustrated further by the following Examples:

### Example 1

### Foodstuffs Composition

A breakfast cereal, for use as a nutritional supplement, contains the following ingredients in each 30 g serving:

| | |
|---|---|
| Vitamin A | 4000 IU |
| Vitamin B₁ | 1 mg |
| Vitamin B₂ | 1mg |
| Vitamin C | 50mg |
| Vitamin D | 400 IU |
| Calcium carbonate | 5mg |
| Lithium carbonate | 20mg |
| Rolled oats | ad 30 g |

### Example 2

### Multi-Vitamin/Mineral Tablet

Multi-vitamin/mineral tablets for daily ingestion each contain the following ingredients:

| | |
|---|---|
| Vitamin A | 4000 IU |
| Vitamin B₁ | 1.5 mg |
| Vitamin B₂ | 1 mg |
| Vitamin B₆ | 1 mg |
| Vitamin B₁₂ | 2 µg |
| Vitamin C | 40 mg |
| Vitamin D | 400 IU |
| Vitamin E | 4 mg |
| Calcium carbonate | 5 mg |
| Lithium citrate | 50 mg |
| Iron (II) carbonate | 10 mg |
| Manganese sulphate | 1 mg |
| Nicotinamide | 15 mg |
| Tableting base | ad 450 mg |

The tablet components are mixed and compressed to form biconvex tablets which are then coated in a conventional manner. If desired lithium gammalinolenate precoated with a gastric Juice resistant release delaying coating (e.g. a Eudragit coating) or incorporated into a matrix of such a coating material and ground to a powder may be incorporated into the multi-vitamin/mineral tablet in place of the lithium carbonate.

### Example 3

### Complete Foodstuff

A complete foodstuff, particularly suited for administration to geriatric or convalescent subjects at a daily dosage of about 500 g/day, is prepared by admixing 50mg of lithium carbonate (or 70 mg lithium gamma linolenate) together with 500g of a complete foodstuffs composition containing all other major essential nutrients.

### Example 4

### Parenteral Alimentation Solution

A solution for lithium-supplemented parenteral alimentation is prepared by dissolving 20mg of lithium citrate (or 1 g lithium linoleate or 1 g lithium linoleate and 0.3 g lithium alpha linolenate or 1 g lithium linoleate, 0.3 g lithium alpha linolenate, 0.3 g lithium gamma linolenate and 0.3 g lithium eicosapentaenoate) in 500ml of a parenteral alimentation solution (e.g. Vamin N or Vamin Glucose, optionally admixed about 13:1 by volume with Ped-El (Vamin and Ped-El are products available from KabiVitrum Ltd, Uxbridge, United Kingdom).

### Example 5

### Complete liquid food

A complete liquid food, for adminstration by enteral tube for example, is prepared for use by geriatrics or convalescents by admixing 50 mg of lithium citrate (or 25 mg of lithium carbonate or 150 mg of lithium gamma linolenate) into 500 g of a conventional complete foodstuffs composition containing all other major essential nutrients.

### Example 6

### Multi-vitamin/mineral tablets

Multi-vitamin and mineral tablets for daily ingestion each contain the following ingredients:

| | |
|---|---|
| Vitamin A | 750 µg |
| Vitamin B₁ | 1.2 mg |
| Vitamin B₂ | 1.6 mg |
| Vitamin B₆ | 1 mg |
| Vitamin B₃ | 2 mg |
| Vitamin B₁₂ | 2 µg |
| Vitamin C | 25 mg |
| Vitamin D | 2.5 µg |
| Vitamin E | 4.5 mg |
| Iron (II) carbonate | 11 mg |
| Calcium carbonate | 4 mg |
| Lithium carbonate | 10 mg |
| Manganese sulphate | 1 mg |
| Nicotinamide | 15 mg |
| Tabletting base | ad 470 mg |

The tablets are prepared analogously to those of Example 2.

### Example 7

### Soup mix

A conventional dried soup mix (single serving) is adjusted by the addition of 5 g lithium carbonate (or 40 g of lithium linoleate, gammalinolenate or eicosapentaenoate).

### Example 8

### Baby food

A baby food (e.g. of the type pre-prepared and packaged in bottles or tins in individual servings) is adjusted by the addition of 2 mg of lithium carbonate (or 4 mg of lithium citrate or 10 mg of lithium gammalinolenate) per 200 kcal calorific value. For a canned vegetable meal, having a calorific value of 89 kcal for a service of 128 g, 0.89 mg of lithium carbonate may thus be uniformly dispersed within the can's contents.

### Example 9

### Injection solutions for addition to parenteral nutrition fluids

Solutions are prepared containing:
(a)

| | |
|---|---|
| lithium linoleate | 1g |
| ethanol/0.9% saline (50/50 by volume) | 10ml |

(b)

| | |
|---|---|
| lithium linoleate | 1g |
| lithium alpha-linolenate | 0.3g |
| ethanol/0.9% saline (50/50 by volume) | 10ml |

(c)

| | |
|---|---|
| lithium linoleate | 1g |
| lithium alpha-linolenate | 0.3g |
| lithium gamma-linolenate | 0.3g |
| lithium eicosapentaenoate | 0.3g |
| ethanol/0.9% saline (50/50 by volume) | 10ml |

These solutions are each filled into vials under aseptic conditions and sealed therein. The contents of such vials may be added to 100ml or 500ml or other sizes of bottles of aqueous fluids for parenteral nutrition which do not already contain essential fatty acids.

In this Example, in place of the essential fatty acid lithium salts specified other lithium salts may instead be used, for example salts of n-6 essential fatty acids such as lithium dihomogamma-linolenate, lithium arachidonate, lithium adrenate and lithium docosapentaenoate(22:5n-6) and salts of the n-3 essential fatty acids such as lithium stearidonate, lithium 20:4n-3, lithium docosapentaenoate (22:5n-3) and lithium docosahexaenoate (22:6n-3).

### Examples 10 to 16

### Nutritional solution supplements

Nutritional solution supplements, for addition to parenteral or enteral nutrition solutions are prepared by dissolving the following materials in water for injections, optionally containing ethanol as up to 50% of the solvent. The solutions are optionally buffered to pH 8.5 to 9.0 and optionally further contain an antioxidant. The supplement solutions are sterile filled into dark glass ampoules which are flushed with and sealed under nitrogen.

| Example No. | 29 | 30 | 31 | 32 | 33 | 34 | 35 |
|---|---|---|---|---|---|---|---|
| Sodium linoleate | | 200 | 250 | 150 | 300 | 500 | 300 |
| Sodium gammalinolenate | | | 100 | 200 | | | 100 |
| Sodium dihomogammalinolenate | | | | | 100 | | |
| Sodium arachidonate | | | 100 | | 100 | | |
| Sodium alphalinolenate | | 250 | 100 | | | | |
| Potassium linoleate | | 200 | | 200 | 200 | | 300 |
| Potassium alphalinolenate | | | | 100 | | 250 | 100 |
| Potassium eicosapentaenoate | | 100 | | | | 100 | 50 |
| Potassium docosahexaenoate | | 100 | | | | 100 | 50 |
| Lithium linoleate | 500 | 200 | 250 | 200 | 200 | | |
| Lithium gammalinolenate | 100 | 250 | | | 100 | 150 | |
| Lithium dihomogammalinolenate | 100 | | 100 | | | 50 | 100 |
| Lithium arachidonate | 100 | | | | | 50 | 50 |
| Lithium alphalinolenate | 250 | 100 | 150 | | | | |
| Lithium eicosapentaenoate | 100 | | 100 | 100 | 100 | | 50 |
| Lithium docosahexaenoate | 100 | | 100 | 100 | 100 | | 50 |
| (The figures are in mg). | | | | | | | |

## Claims

1. The use of a physiologically tolerable lithium compound for the manufacture of a nutritional supplement for use in combatting conditions associated with lithium deficiency in humans, said nutritional supplement being in the form of a complete foodstuff or a multi-vitamin/multi-mineral preparation.

2. Use as claimed in claim 1 of a lithium salt selected from lithium carbonate, lithium citrate, lithium chloride, lithium succinate, lithium sulphate, lithium salicylate, lithium acetylsalicylate and lithium orotate.

3. Use as claimed in either one of claims 1 and 2 for the manufacture of a nutritional supplement in the form of a parenteral or enteral alimentation solution.

4. Use as claimed in any one of claims 1 to 3 for the manufacture of a nutritional supplement in the form of a sterile composition consisting essentially of a physiologically tolerable lithium compound together with four or more vitamins or minerals, and optionally together with at least one physiologically acceptable carrier or excipient.

5. Use as claimed in claim 4 wherein said supplement comprises four or more essential vitamins or minerals selected from the group consisting of vitamins A, B₁, B₂, B₃, B₆, B₁₂, C, D and E and physiologically tolerable calcium, copper, zinc, manganese and iron compounds.

6. Use as claimed in claim 4 or claim 5 wherein said supplement comprises 1 ppb to 10% by weight lithium.

7. Use as claimed in any one of claims 1 to 6 wherein said supplement comprises one or more essential fatty acids or physiologically tolerable salts thereof.

8. Use as claimed in claim 7 wherein said supplement contains at least one lithium, sodium or potassium essential fatty acid salt.

9. Use as claimed in either one of claims 7 and 8 wherein said supplement contains at least one n-3 essential fatty acid or salt thereof and at least one n-6 essential fatty acid or salt thereof.

## Patentansprüche

1. Verwendung einer physiologisch verträglichen Lithium-Verbindung zur Herstellung eines Nahrungszusatzes zur Bekämpfung von Lithiummangelzuständen beim Menschen, wobei der Nahrungszusatz in Form eines vollständigen Nahrungsmittels oder einer Multivitamin-/Multimineral-Präparation vorliegt.

2. Verwendung nach Anspruch 1 eines Lithium-Salzes, ausgewählt aus Lithiumcarbonat, Lithiumcitrat, Lithiumchlorid, Lithiumsuccinat, Lithiumsulfat, Lithiumsalicylat, Lithiumacetylsalicylat und Lithiumorotat.

3. Verwendung gemäß einem der Ansprüche 1 und 2 zur Herstellung eines Nahrungszusatzes in Form einer parenteralen oder enteralen Nährlösung.

4. Verwendung gemäß einem der Ansprüche 1 bis 3 zur Herstellung eines Nahrungszusatzes in Form einer sterilen Zusammensetzung, im wesentlichen bestehend aus einer physiologisch verträglichen Lithium-Verbindung, zusammen mit vier oder mehr Vitaminen oder Mineralien und gegebenenfalls zusammen mit mindestens einem physiologisch akzeptablen Träger oder Verdünnungsmittel.

5. Verwendung gemäß Anspruch 4, wobei der Zusatz vier oder mehr essentielle Vitamine oder Mineralien umfaßt, ausgewählt aus der aus den Vitaminen A, B₁, B₂, B₃, B₆, B₁₂, C, D und E und physiologisch verträglichen Calcium-, Kupfer-, Zink-, Mangan- und Eisenverbindungen bestehenden Gruppe.

6. Verwendung gemäß Anspruch 4 oder Anspruch 5, wobei der Zusatz 1 ppb bis 10 Gew.-% Lithium umfaßt.

7. Verwendung gemäß einem der Ansprüche 1 bis 6, wobei der Zusatz eine oder mehrere essentielle Fettsäuren oder deren physiologisch verträgliche Salze umfaßt.

8. Verwendung gemäß Anspruch 7, wobei der Zusatz mindestens ein essentielles Lithium-, Natrium- oder Kaliumsalz einer Fettsäure enthält.

9. Verwendung gemäß einem der Ansprüche 7 und 8, wobei der Zusatz mindestens eine n-3 essentielle Fettsäure oder deren Salz und mindestens eine n-6 essentielle Fettsäure oder deren Salz enthält.

## Revendications

1. Utilisation d'un composé de lithium physiologiquement tolérable en vue de la fabrication d'un supplément nutritif à utiliser pour combattre des états associés à une déficience en lithium d'êtres humains, ledit supplément nutritif se présentant sous la forme d'un aliment complet ou d'une préparation multivitaminée/multiminérale.

2. Utilisation suivant la revendication 1 d'un sel de lithium choisi parmi le carbonate de lithium, le citrate de lithium, le chlorure de lithium, le succinate de lithium, le sulfate de lithium, la salicylate de lithium, l'acétylsalicylate de lithium et l'orotate de lithium.

3. Utilisation suivant l'une quelconque des revendications 1 et 2 en vue de la fabrication d'un supplément nutritif qui se présente sous la forme d'une solution alimentaire parentérale ou entérale.

4. Utilisation suivant l'une quelconque des revendications 1 à 3 en vue de la fabrication d'un supplément nutritif qui se présente sous la forme d'une composition stérile essentiellement constituée d'un composé de lithium physiologiquement tolérable, en même temps que de quatre ou plus de quatre vitamines ou substances minérales, et éventuellement en même temps que d'au moins un véhicule ou excipient physiologiquement compatible.

5. Utilisation suivant la revendication 4, caractérisée en ce que le supplément en question est constitué de quatre ou de plus de quatre substances minérales ou vitamines essentielles, que l'on choisit dans le groupe formé par les vitamines A, B₁, B₂, B₃, B₆, B₁₂, C, D et E et des composés physiologiquement tolérables du calcium, du cuivre, du zinc, du manganèse et du fer.

6. Utilisation suivant la revendication 4 ou la revendication 5, caractérisée en ce que le supplément comprend 1 partie par milliard à 10% en poids de lithium.

7. Utilisation suivant l'une quelconque des revendications 1 à 6, caractérisée en ce que le supplément en question comprend un ou plusieurs acides gras essentiels ou sels de ceux-ci physiologiquement tolérables.

8. Utilisation suivant la revendication 7, caractérisée en ce que le supplément en question contient au moins un sel de lithium, de sodium ou de potassium d'un acide gras essentiel.

9. Utilisation suivant l'une quelconque des revendications 7 et 8, caractérisée en ce que le supplément concerné contient au moins un acide gras essentiel n-3 ou un sel de celui-ci et au moins un acide gras essentiel n-6 ou un sel de celui-ci.
